# EUROPÄISCHE PATENTSCHRIFT

(11) **EP 3 795 358 B1**
(45) Veröffentlichungstag und Bekanntmachung des Hinweises auf die Patenterteilung: **25.03.2026**
(21) Anmeldenummer: 19198629.8
(22) Anmeldetag: 20.09.2019
(51) Int. Cl.: A61M 37/00, B33Y 10/00, B33Y 80/00, B22F 5/10, B22F 10/16, B29C 64/10

(54) **MIKRONADEL UND VERFAHREN ZUR HERSTELLUNG EINER MIKRONADEL**
MICRONEEDLE AND METHOD FOR MANUFACTURING SAME
MICRO AIGUILLE ET PROCÉDÉ DE FABRICATION D'UNE MICRO AIGUILLE

(43) Veröffentlichungstag der Anmeldung: 24.03.2021
(73) Patentinhaber: Axenoll Life Sciences AG, 8001 Zürich (CH)
(72) Erfinder: Vasic, Srdan, 9126 Zumikon (CH); Schmitt, Rafael, 9126 Zumikon (CH)
(74) Vertreter: Wenzel Nemetzade Warthmüller Patentanwälte Part mbB

(56) Entgegenhaltungen:
- WO-A1-2016/149152
- WO-A1-2019/136133
- CN-A- 109 125 912
- CN-A- 109 747 149
- US-A1- 2018 177 990

## Beschreibung

Die vorliegende Erfindung betrifft ein Verfahren zur Herstellung einer Mikronadel, insbesondere zur transdermalen und/oder intradermalen Wirkstoffverabreichung. Ebenso betrifft die vorliegende Erfindung ein Verfahren zur Herstellung einer Mikronadelvorrichtung.

Transdermale therapeutische Systeme oder transdermale Pflaster können Wirkstoffe nach der Permeation der Haut systemisch zur Verfügung stellen. Es existieren jedoch Wirkstoffe, die durch das bloße Aufbringen auf der Haut nicht durch den Körper aufgenommen werden können. Insbesondere können bestimmte Arzneistoffe die Hauptdiffusionsbarriere der Haut, das sogenannte Stratum corneum beziehungsweise die Hornzellschicht, nicht überwinden. Aus diesem Grund sind sogenannte Mikronadelpflaster beziehungsweise Micro Array Patches entwickelt worden. Solche Mikronadelpflaster beziehungsweise Micro Array Patches weisen eine Vielzahl sehr kleiner Nadeln auf, die die oberen Hautschichten durchdringen und dadurch eine verbesserte Wirkstoffverabreichung ermöglichen.

Zur Herstellung von Mikronadeln kann beispielsweise ein Mikrogussverfahren (micro molding), ein Lithografieverfahren (soft lithography oder drawing lithography) zum Einsatz kommen. Ebenso bekannt ist das sogenannte Droplet Born Airblowing sowie das Electrospun Pillar Array Verfahren. Diese Verfahren sind nur eingeschränkt zur Herstellung größerer Stückzahlen geeignet. Zudem besteht die Gefahr einer Materialbelastung durch hohe Verarbeitungstemperaturen. In Gussverfahren kann es ferner zu einem hohen Materialverbrauch und damit auch zu Wirkstoffverschwendung aufgrund von Angussstrukturen kommen. Schließlich bestehen Einschränkungen hinsichtlich der Trägermaterialwahl.

Der Stand der Technik in der Druckschrift CN 109125912 A betrifft ein 3D-gedrucktes Mikronadelpflaster zur Regulierung des Blutzuckerspiegels, das aus einem biokompatiblen lichtempfindlichen Material hergestellt ist. Das Mikronadelpflaster umfasst im Wesentlichen zwei Teile, nämlich ein Substrat sowie eine Mikronadelanordnung, wobei die Mikronadelanordnung eine Vielzahl von konischen Mikronadeln enthält.

Die Druckschrift WO 2019/136133 A1 betrifft eine Mikronadel, die einen länglichen Körper mit einem abnehmbaren Abschnitt umfasst, wobei der abnehmbare Abschnitt ein therapeutisches Mittel umfasst.

Die Druckschrift WO 2016/149152 A1 betrifft eine Mikronadelvorrichtung, umfassend eine Unterlage und eine Vielzahl von biokompatiblen Mikronadeln, die von der Unterlage abstehen. Die Mikronadeln weisen dabei auf: (i) eine gekrümmte, diskontinuierliche, hinterschnittene und/oder perforierte Seitenwand; (ii) eine Seitenwand, die eine zerbrechliche Stütze umfasst; und/oder (iii) einen Querschnitt, der nicht kreisförmig und nicht polygonal ist, und/oder wobei die Mikronadeln gestuft sind und wobei die Mikronadeln eine Querschnittsbreite haben, die in beiden Dimensionen entlang mindestens eines Teils ihrer Länge variiert.

Die Druckschrift US 2018/177990 A1 betrifft ein Mikronadel-Array, umfassend einen Film mit ersten und zweiten, nach außen weisenden Hauptoberflächen, wobei die erste nach außen weisende Hauptoberfläche eine Vielzahl von Mikronadeln aufweist, die die Hornschicht durchdringen, und wobei die Vielzahl von Mikronadeln eine Vielzahl von ersten Mikronadeln mit einer ersten Wirkstoff und eine Vielzahl zweiter Mikronadeln mit einem zweiten Wirkstoff aufweist.

Der Stand der Technik in der Druckschrift CN 109 747 149 A betrifft ein Verfahren zur Herstellung von Mikronadeln, bei dem eine kegelförmige Spitzenstruktur in einer Schicht aus gedrucktem Material durch Steuerung der Belichtungszeit eines Druckstrahls unter Verwendung eines DPD-basierten 3D-Druckers freigelegt wird.

Vor dem oben dargelegten Hintergrund bestand die Aufgabe der vorliegenden Erfindung darin, ein Verfahren zur Herstellung einer Mikronadel anzugeben, mit dem bei begrenztem Aufwand und größerer Flexibilität beziehungsweise breiteren Anwendungsmöglichkeiten in hohen Stückzahlen gefertigt werden kann. Ebenso bestand die Aufgabe darin, ein Verfahren zur Herstellung einer Mikronadelvorrichtung anzugeben.

In Bezug auf ein Verfahren zur Herstellung einer Mikronadel ist diese Aufgabe durch den Gegenstand des Anspruchs 1 gelöst worden. Eine erfindungsgemäßes Verfahren zur Herstellung einer Mikronadelvorrichtung ist Gegenstand von Anspruch 14. Vorteilhafte Ausgestaltungen sind in den abhängigen Ansprüchen angegeben.

Ein erster Aspekt der vorliegenden Erfindung betrifft ein Verfahren zur Herstellung einer Mikronadel, insbesondere zur transdermalen und/oder intradermalen Wirkstoffverabreichung, bei dem eine Tragstruktur bereitgestellt wird und bei dem wenigstens eine an der Tragstruktur angeordnete Nadelstruktur zur Durchdringung der Hornzellschicht menschlicher und/oder tierischer Haut schichtweise durch 3D-Siebdruck erzeugt wird, wobei zwischen einzelnen Schritten zur schichtweisen Erzeugung der Nadelstruktur Trocknungsschritte erfolgen, durch die eine Trocknung der jeweils vorgedruckten Schicht gewährleistet wird.

Eine erfindungsgemäß hergestellte Mikronadel eignet sich insbesondere zur transdermalen und/oder intradermalen Wirkstoffverabreichung. Durch eine solche Mikronadel kann also eine Wirkstoffverabreichung durch die Haut hindurch und/oder in die Haut hinein erfolgen. Hierdurch kann eine größere Flexibilität bei der Wirkstoffverabreichung erzielt werden.

Eine erfindungsgemäß hergestellte Mikronadel weist hierzu die Tragstruktur und wenigstens die eine an der Tragstruktur angeordnete Nadelstruktur zur Durchdringung der Hornzellschicht menschlicher und/oder tierischer Haut auf. Erfindungsgemäß wird zumindest die Nadelstruktur durch 3D-Siebdruck erzeugt.

Die Nadelstruktur kann insbesondere fest an der Tragstruktur angeordnet werden. Die Tragstruktur kann demnach zum Halten der Nadelstruktur ausgebildet sein und damit eine Handhabung der Nadelstruktur für den Einsatz der jeweiligen Wirkstoffverabreichung vereinfachen.

Die Erzeugung der Nadelstruktur durch 3D-Siebdruck ermöglicht einerseits ein höheres Maß an Flexibilität hinsichtlich der Materialzusammensetzung sowie Formgebung der Nadelstruktur. Gleichzeitig kann durch den Einsatz des 3D-Siebdrucks mit nur geringem Aufwand eine große Stückzahl von Mikronadeln beziehungsweise Nadelstrukturen bereitgestellt werden. Durch den Einsatz des 3D-Siebdrucks kann zudem die Erzeugung einer Nadelstruktur mit nur geringer Material- beziehungsweise Temperaturbelastung realisiert werden. Beschränkungen hinsichtlich der Materialauswahl und/oder im Hinblick auf die zu verarbeitenden Wirkstoffe lassen sich auf diese Weise verringern.

Unter dreidimensionalem Siebdruck kann vorliegend in besonders bevorzugter Weise ein additives Fertigungsverfahren verstanden werden, bei dem eine pulverbasierte Suspension mithilfe einer Rakel durch eine feste Druckmaske, insbesondere ein Drucksieb und/oder eine Druckschablone, auf ein Substrat übertragen und getrocknet wird. Diese Vorgehensweise kann mehrmals wiederholt werden, bis die jeweils gewünschte Bauteilhöhe oder Bauteilform erreicht ist. Hierdurch kann ein Siebdruckwerkstück entstehen.

Unter Siebdruckwerkstück können vorliegend in bevorzugter Weise Werkstücke verstanden werden, die einem Trocknungs- und/ oder Sinterschritt zu unterziehen sind, beziehungsweise unterzogen worden sind. Dies betrifft insbesondere Werkstücke aus einem Metall, einer Keramik, einem Glasmaterial und/oder einem Polymermaterial. Auch Druckerzeugnisse aus Polymermaterialien und/oder aus Zellulose enthaltenden beziehungsweise aus Zellulose bestehenden Materialien können durch die Bezeichnung "dreidimensionales Siebdruckwerkstück" mitumfasst sein. Insbesondere besteht auch die Möglichkeit, gedruckte Werkstücklagen aus Polymermaterial einem Sinterschritt zu unterziehen. Unter Siebdruckwerkstück können vorliegend auch Werkstücke verstanden werden, die aus nichtsinterfähigen Materialien beziehungsweise ohne Sinterschritt hergestellt sind.

Im Sinne der vorliegenden Erfindung handelt es sich bei einem Siebdruckwerkstück insbesondere um ein Werkstück, das zumindest teilweise mittels dreidimensionalen Siebdrucks erzeugt ist.

Gemäß einer bevorzugten Ausgestaltung kann auch die Tragstruktur durch additive Fertigung, insbesondere 3D-Siebdruck, erzeugt werden. Auf diese Weise lässt sich die Fertigungsflexibilität weiter erhöhen. Die gesamte Mikronadel kann auf diese Weise mit nur geringerem Aufwand und in großen Stückzahlen bereitgestellt werden. Ferner lassen sich auf diese Weise die Nadelstruktur und die Tragstruktur durch dasselbe Material beziehungsweise dasselbe Grundmaterial herstellen, wodurch der Fertigungsaufwand weiter verringert werden kann. Dementsprechend kann die gesamte Mikronadel durch additive Fertigung, insbesondere 3D-Siebdruck, erzeugt werden.

Gemäß einer weiter bevorzugten Ausgestaltung können die Nadelstruktur und die Tragstruktur einstückig ausgebildet werden. Durch eine einstückige Ausbildung der Nadelstruktur und der Tragstruktur kann insbesondere sichergestellt werden, dass die Nadelstruktur mit ausreichend hoher Festigkeit an der Tragstruktur angeordnet ist und somit ein unerwünschtes Ablösen vermieden wird. Ebenso ist es möglich, dass die Nadelstruktur und die Tragstruktur durch eine ununterbrochene Verfahrensabfolge hergestellt werden. Die Herstellung der Nadelstruktur und der Tragstruktur durch eine ununterbrochene Verfahrensabfolge kann insbesondere durch den Einsatz des dreidimensionalen Siebdrucks erfolgen. Hierzu kann beispielsweise die Tragstruktur durch eine Schicht oder mehrere Schichten erzeugt werden und auf der Tragstruktur durch weitere Druckabfolgen die Nadelstruktur aufgetragen werden. Zwischen den einzelnen Druckschritten erfolgt eine Trocknung des gedruckten Materials. Im Anschluss an die Druckabfolgen kann eine thermische Verfestigung vorgenommen werden, beispielsweise durch Einsatz von UV Licht.

Gemäß einer weiter bevorzugten Ausgestaltung kann die Tragstruktur separat von der Nadelstruktur erzeugt werden. Es besteht die Möglichkeit, dass die Nadelstruktur mittels 3D-Siebdrucks an der Tragstruktur angeordnet und/oder befestigt wird. Die Nadelstruktur kann dementsprechend schichtweise auf die separat erzeugte Tragstruktur aufgetragen werden, nämlich durch den schichtweisen Aufbau im 3D-Siebdruckverfahren.

Es ist insbesondere möglich, dass die Tragstruktur anders als die Nadelstruktur erzeugt wird, nämlich ohne Einsatz additiver Fertigung oder 3D-Siebdruck. Auf eine derart alternativ erzeugte Tragstruktur kann dann eine Nadelstruktur durch 3D-Siebdruck aufgebracht und hierdurch mit der Tragstruktur verbunden werden. Je nach Art der Erzeugung der Tragstruktur kann der Fertigungsaufwand auf diese Weise weiter verringert werden.

Gemäß einer weiter bevorzugten Ausgestaltung kann die Nadelstruktur zumindest abschnittsweise zylindrisch ausgebildet werden und/oder mit einem entlang ihrer Längserstreckung zumindest abschnittsweise gleichbleibenden und/oder kreisrunden Querschnitt ausgebildet werden. Ferner ist es möglich, dass die gesamte Nadelstruktur zylindrisch ausgebildet wird und/oder mit einem entlang ihrer Längserstreckung gleichbleibenden und/oder kreisrunden Querschnitt ausgebildet werden. Eine derartige geometrische Ausgestaltung lässt sich im Wege der additiven Fertigung mit nur geringem Aufwand erzeugen, wodurch die Herstellkosten, insbesondere bei großen Stückzahlen, verringert werden können.

Ein kreisrunder Querschnitt kann insbesondere durch Herstellschritte mit begrenzter Komplexität erzeugt werden. Die in Längserstreckung zumindest abschnittsweise gleichbleibende Querschnittsform ermöglicht beispielsweise die Beibehaltung von Fertigungsparametern entlang der Längserstreckung beziehungsweise in der Erzeugung mehrerer aufeinander angeordneter Schichten.

Anstelle eines runden beziehungsweise kreisrunden Querschnitts können auch andere Querschnittsformen realisiert werden. Beispielsweise kann die Nadelstruktur zumindest abschnittsweise mit einem ovalen, rechteckigen, insbesondere quadratischen, oder dreieckigen, fünfeckigen oder sechseckigen Querschnitt ausgebildet werden. Auch derartige Querschnittsformen können zumindest abschnittsweise gleichbleibend ausgebildet sein, also in Längsrichtung beziehungsweise Längserstreckung der Nadelstruktur gleichbleibend ausgebildet werden.

Es kann weiter von Vorteil sein, wenn die Nadelstruktur mit einem entlang ihrer Längserstreckung sich verändernden Querschnitt ausgebildet wird und/oder mit unterschiedlichen Querschnittsgrößen und/oder gleichbleibenden oder sich verändernden Querschnittsformen ausgebildet wird. Die Flexibilität der Formgebung kann auf diese Weise weiter verbessert werden. Unterschiedliche Abschnitte der Nadelstruktur entlang der Längserstreckung lassen sich je nach funktionalen Erfordernissen hinsichtlich der äußeren Abmessungen sowie der äußeren Formgebung gezielt ausgestalten, beispielsweise im Hinblick auf die Durchdringung der Hornzellschicht menschlicher oder tierischer Haut beziehungsweise der Verabreichung oder Abgabe des jeweils gewünschten Wirkstoffs in oder durch die jeweilige Haut.

In besonders bevorzugter Weise kann die Nadelstruktur entlang ihrer Längserstreckung gestuft ausgebildet werden und/oder mit einem zwischen wenigstens zwei Stufen gleichbleibenden Querschnitt, insbesondere mit einer gleichbleibenden Querschnittsform und/oder Querschnittsgröße ausgebildet werden. Durch eine solche Ausgestaltung der Nadelstruktur kann einerseits eine Veränderung der Nadelstruktur entlang der Längserstreckung realisiert werden, wobei gleichzeitig der Aufwand zur Erzeugung der jeweiligen Veränderungen begrenzt werden kann. So lässt sich auf diese Weise beispielsweise ein verhältnismäßig kleiner Spitzenbereich der Nadelstruktur erzeugen, durch den die jeweilige Hornzellschicht einfach durchdrungen werden kann. Gleichzeitig kann der an die Tragstruktur angrenzende Bereich der Nadelstruktur mit größerer Dicke beziehungsweise größeren Außenabmessungen erzeugt werden, durch den eine gute Verbindung mit der Tragstruktur und auch eine größere Wirkstoffverabreichung ermöglicht wird. Die Effektivität des Einsatzes der jeweiligen Mikronadel kann hierdurch verbessert werden.

Gemäß einer weiter bevorzugten Ausgestaltung kann die Nadelstruktur entlang ihrer Längserstreckung zumindest abschnittsweise mit einem Querschnittsdurchmesser von mindestens 30 µm, bevorzugt von mindestens 50 µm, bevorzugt von mindestens 70 µm, weiter bevorzugt von mindestens 80 µm oder von mehr als 90 µm, insbesondere mehr als 100 µm, noch weiter bevorzugt von mehr als 150 µm, noch weiter bevorzugt von mehr als 200 µm, noch weiter bevorzugt von mehr als 250 µm oder noch weiter bevorzugt von mehr als 300 µm ausgebildet werden. Durch eine derartige Dimensionierung der Abmessungen der Nadelstruktur kann einerseits eine ausreichende mechanische Steifigkeit gewährleistet werden, so dass die Durchdringung der Hornzellschicht menschlicher und/oder tierischer Haut mit einem hohen Maß an Sicherheit gewährleistet werden kann. Gleichzeitig kann durch eine derartige Dimensionierung ein ausreichend hoher Wirkstoffgehalt innerhalb der Nadelstruktur oder an der Nadelstruktur gewährleistet werden.

Gemäß einer weiter bevorzugten Ausgestaltung kann die Nadelstruktur entlang ihrer Längserstreckung zumindest abschnittsweise mit einem Querschnittsdurchmesser von weniger als 300 µm, bevorzugt von weniger als 250 µm, bevorzugt von weniger als 200 µm, weiter bevorzugt von weniger als 150 µm oder von weniger als 100 µm oder von weniger als 90 µm oder von weniger als 80 µm ausgebildet werden. Durch eine derartige geometrische Ausgestaltung der Nadelstruktur kann ein sicheres und schmerzarmes beziehungsweise schmerzfreies Eindringen der Nadelstruktur durch die Hornzellschicht menschlicher und/oder tierischer Haut gewährleistet werden. Insbesondere im Bereich der Spitze der Nadelstruktur, die an einem von der Tragstruktur abgewandten Ende der Nadelstruktur ausgebildet ist, können kleine Querschnittsdurchmesser von Vorteil sein. Die Durchdringung der Hornzellschicht kann mit verhältnismäßig kleinen Querschnittsdurchmessern einfach bewerkstelligt werden.

Für den Fall einer eckigen beziehungsweise rechteckigen Querschnittsform, können sich die voranstehenden Maßangaben auf die Länge einer Diagonale beziehen. Allgemein können sich die voranstehenden Maßangaben auf den größtmöglichen Abstand zwischen zwei Punkten auf dem Außenumfang einer Querschnittsebene beziehen. Hierbei kann es sich beispielsweise um die Länge einer Diagonale eines rechteckförmigen Querschnitts handeln.

Es kann weiter von Vorteil sein, wenn die Nadelstruktur mit einer Gesamtlänge von mindestens 200 µm, mindestens 300 µm, mindestens 400 µm, mindestens 500 µm, mindestens 600 µm oder mindestens 700 µm ausgebildet wird. Durch eine derartige Längendimensionierung kann ein sicheres Durchdringen der Hornzellschicht menschlicher und/oder tierischer Haut durch die Nadelstruktur gewährleistet werden. Ebenso ist es möglich, dass die Nadelstruktur mit einer Gesamtlänge von weniger als 1000 µm, weniger als 900 µm, weniger als 800 µm, weniger als 700 µm, weniger als 600 µm, weniger als 500 µm oder weniger als 400 µm ausgebildet wird. Hierdurch kann ein zu tiefes Eindringen der Nadelstruktur in das jeweilige Gewebe sowie auch eine unerwünschte Verformung der Nadelstruktur vermieden werden. Durch eine geeignete Begrenzung der Länge der Nadelstruktur kann insbesondere die mechanische Stabilität begünstigt und hierdurch wiederum die jeweils gewünschte Durchdringung der Hornzellschicht mit einem hohen Maß an Sicherheit gewährleistet werden.

Gemäß einer weiter bevorzugten Ausgestaltung kann die Nadelstruktur mit zumindest einem sich in Längsrichtung zwischen zwei Stufen erstreckenden Nadelstrukturabschnitt mit einer Länge von weniger als 200 µm, weniger als 150 µm, weniger als 100 µm oder weniger als 50 µm ausgebildet werden. Durch die Begrenzung der Länge eines solchen Nadelstrukturabschnitts können jeweils gewünschte Variationen der Querschnittsdimensionierung beziehungsweise Querschnittsform an weiteren Nadelstrukturabschnitten beziehungsweise entlang weiterer Nadelstrukturabschnitte vorgenommen werden.

Gemäß einer weiter bevorzugten Ausgestaltung kann die Nadelstruktur mit wenigstens einem sich in Längserstreckung zwischen zwei Stufen erstreckenden Nadelstrukturabschnitt mit einer Länge von mindestens 20 µm, mindestens 50 µm, mindestens 100 µm, mindestens 150 µm, mindestens 200 µm oder mindestens 250 µm ausgebildet werden. Eine solche Dimensionierung der Nadelstruktur zwischen zwei benachbarten Stufen ermöglicht die Fertigung der Nadelstruktur mit verhältnismäßig geringem Aufwand. Insbesondere kann zwischen zwei benachbarten Stufen die Querschnittsform beziehungsweise Querschnittsdimensionierung beibehalten werden, so dass für die Erzeugung des jeweiligen Nadelstrukturabschnitts die Fertigungsparameter beibehalten werden können. Beispielsweise können beim Einsatz des dreidimensionalen Siebdrucks für die Erzeugung des jeweiligen Nadelstrukturabschnitts dieselben Drucksiebe beziehungsweise Druckschablonen eingesetzt werden. Mehrere Druckschichten können demnach durch dasselbe Drucksieb beziehungsweise durch dieselbe Druckschablone erzeugt werden, so dass der Handhabungsaufwand in der Fertigung des jeweiligen Nadelstrukturabschnitts auf ein geringes Maß reduziert werden kann.

Gemäß einer noch weiter bevorzugten Ausgestaltung des erfindungsgemäßen Verfahrens kann die Nadelstruktur mit wenigstens einem Wirkstoff oder auch mit mehreren Wirkstoffe ausgebildet werden. Ein in der Nadelstruktur vorgesehener Wirkstoff kann mit hoher Sicherheit nach der Durchdringung der Hornzellschicht menschlicher und/oder tierischer Haut in dem jeweiligen Organismus freigesetzt und somit systemisch zur Verfügung gestellt werden. Es ist ebenso möglich, dass die Nadelstruktur frei von Wirkstoffen ausgestaltet wird und lediglich zur Perforation der Hornzellschicht menschlicher und tierischer Haut geeignet ist. Nach der jeweiligen Perforation kann durch ein Pflaster der jeweilige Wirkstoff durch die perforierten Stellen verabreicht werden.

In weiter bevorzugter Weise kann die Nadelstruktur zur Wirkstoffverabreichung durch Materialauflösung ausgebildet werden. Durch Materialauflösung kann eine besonders präzise Wirkstoffverabreichung beziehungsweise Dosierung gewährleistet werden. Sofern die jeweilige Nadelstruktur zur vollständigen Materialauflösung ausgebildet wird, ist die nachträgliche Entfernung der Nadelstruktur aus dem jeweiligen Gewebe entbehrlich. Die Anwenderfreundlichkeit wird damit verbessert.

Gemäß einer weiter bevorzugten Ausgestaltung kann die Nadelstruktur entlang ihrer Längserstreckung mit unterschiedlichen Wirkstoffdichten ausgebildet werden. Ebenso ist es möglich, dass die Nadelstruktur entlang ihrer Längserstreckung mit unterschiedlichen Wirkstoffen ausgebildet wird beziehungsweise die jeweiligen Wirkstoffe entlang der Längserstreckung in unterschiedlicher Dichte beziehungsweise Menge vorgesehen werden. Die Wirkstoffverabreichung in unterschiedlichen Schichten der Haut kann somit präzise eingestellt beziehungsweise gesteuert werden. Unterschiedliche Wirkstoffe lassen sich somit in unterschiedlichen Ebenen beziehungsweise Schichten der Haut verabreichen, wodurch die Funktionalität der Mikronadel weiter verbessert wird.

Durch unterschiedliche Wirkstoffdichten entlang der Längserstreckung der Nadelstruktur lassen sich zudem zeitliche Profile der Wirkstoffverabreichung in geeigneter Weise steuern beziehungsweise regulieren. Je nachdem, in welcher Gewebetiefe eine Freisetzung der Wirkstoffe erfolgt, kann eine unterschiedlich schnelle systemische Bereitstellung des jeweiligen Wirkstoffs erfolgen.

Eine Wirkstoff- beziehungsweise Wirkstoffdichtenvariation entlang der Längserstreckung der Nadelstruktur kann mit besonders geringem Aufwand im Wege des dreidimensionalen Siebdrucks realisiert werden. Hierzu können unterschiedliche Schichten der Nadelstruktur mittels unterschiedlicher Druckpasten beziehungsweise unterschiedlich zusammengesetzter Druckpasten erzeugt werden. Auf diese Weise kann mit nur geringem Aufwand ein Wirkstoffgradient beziehungsweise eine Wirkstoffvariation entlang der Längserstreckung der Nadelstruktur erzielt werden.

Es kann weiter von Vorteil sein, wenn die Nadelstruktur mit einer zur Auflösung ausgebildeten Beschichtung mit wenigstens einem Wirkstoff ausgebildet wird. Es kann dementsprechend eine Nadelstruktur mit einem Nadelkern und darauf ausgebildeter Beschichtung bereitgestellt werden. Dabei kann die Beschichtung zur Auflösung in lebendem Gewebe ausgebildet sein und der Kern der Nadelstruktur nach der Auflösung der Beschichtung erhalten bleiben. Der Kern der Nadelstruktur kann gemeinsam mit der Beschichtung der Nadelstruktur mittels additiver Fertigung, insbesondere 3D-Siebdruck erzeugt werden. Ebenso ist es möglich, dass auch der Kern der Nadelstruktur zur Auflösung in lebendem Gewebe ausgebildet wird, insbesondere in einer definierten zeitlichen Abfolge nach der Beschichtung.

In weiter bevorzugter Ausgestaltung kann die Nadelstruktur mit einem Hohlraum mit wenigstens einem darin angeordneten Wirkstoff ausgebildet werden. Nach Durchdringung einer Hornzellschicht der menschlichen und/oder tierischen Haut kann der jeweilige Wirkstoff aus dem Hohlraum der Nadelstruktur freigesetzt und somit systemisch innerhalb des jeweiligen Organismus zur Verfügung gestellt werden. Dementsprechend kann die Nadelstruktur zur Wirkstoffverabreichung aus einem Hohlraum der Nadelstruktur ausgebildet werden. Die Wirkstoffverabreichung kann somit unabhängig von der Auflösung der Nadelstruktur beziehungsweise der vollständigen Auflösung der Nadelstruktur oder einer jeweiligen Beschichtung erfolgen und somit in verhältnismäßig kurzer Zeit realisiert werden.

Es kann weiter von Vorteil sein, wenn die Nadelstruktur und/oder die Tragstruktur aus Polyvinylpyrrolidon (PVP) oder einem Polyvinylpyrrolidon (PVP) enthaltenden Werkstoff erzeugt wird. Allgemein kann die Nadelstruktur und/oder die Tragstruktur aus einem Polymer bestehen und/oder aus einem Polymer enthaltenden Werkstoff gefertigt sein. Es besteht ferner die Möglichkeit, dass die Nadelstruktur und/oder die Tragstruktur aus einer Mehrzahl von Werkstoffbestandteilen beziehungsweise Materialbestandteilen erzeugt wird, beispielsweise Glycerin, Polysorbat 80, Trehalose, di-Natriumhydrogenphosphat-Dodecahydrat, di-Natriumhydrogenphosphat-Monohydrat und/oder destilliertes Wasser (oder allgemein "purified water") beziehungsweise Lösungsmittel. In weiter bevorzugter Weise kann der zur Erzeugung der Nadelstruktur und/oder der Tragstruktur eingesetzte Werkstoff viskositätserhöhende Bestandteile enthalten, insbesondere um die Verarbeitbarkeit des Werkstoffs im Wege der additiven Fertigung, insbesondere des 3D-Siebdrucks, zu verbessern.

In weiter bevorzugter Weise kann für die Erzeugung der Nadelstruktur und/oder der Tragstruktur ein Werkstoff eingesetzt werden, der zumindest eine der folgenden Bestandteile, insbesondere als Matrixwerkstoff, enthält: Hyaloronsäure, Carboxymethylcellulosen, Polyvinylalkohol (PVA), Polyvinylpyrrolidon (PVO), PVM/MA Copolymer, Poly(lactid-co-glycolid) (PLGA), Polylactide (PLA) und/oder Polyglycolsäure (PGA). Durch den Einsatz derartiger Bestandteile kann die Fertigungsflexibilität und auch die Einsatzflexibilität der jeweiligen Mikronadel weiter verbessert werden.

Ein noch weiterer Aspekt der vorliegenden Erfindung betrifft ein Verfahren zur Herstellung reine Mikronadelvorrichtung, insbesondere zur transdermalen und/oder intradermalen Wirkstoffverabreichung, bei dem eine Mehrzahl von Mikronadeln gemäß dem voranstehend beschriebenen Verfahren erzeugt werden. Dabei können die Mikronadeln in bevorzugter Weise als sogenanntes Nadelarray ausgebildet werden. Unter einem Nadelarray soll vorliegend eine regelmäßige Anordnung oder auch eine unregelmäßige Anordnung von Mikronadeln entlang eines räumlich abgegrenzten Bereichs betreffen. Ein Nadelarray kann beispielsweise kreisförmig oder rechteckförmig ausgebildet sein und/oder eine definierte Anzahl von Mikronadeln enthalten.

Gemäß einer weiter bevorzugten Ausgestaltung können die Tragabschnitte der Mikronadeln einstückig miteinander ausgebildet oder zu einer Gesamttragstruktur verbunden werden. Die Mikronadeln können somit eine definierte Anordnung relativ zueinander aufweisen und auch beibehalten, so dass die Handhabbarkeit sowie Anwenderfreundlichkeit der Mikronadelvorrichtung verbessert wird.

In weiter bevorzugter Weise können zumindest zwei benachbarte Mikronadeln einer Mikronadelvorrichtung einen Abstand von mindestens 100 µm, mindestens 200 µm, mindestens 300 µm, mindestens 350 µm, mindestens 400 µm, mindestens 500 µm, mindestens 600 µm, mindestens 700 µm, mindestens 800 µm oder mindestens 1000 µm zueinander aufweisen. Ebenso ist es möglich, dass wenigstens zwei zueinander benachbarte Nadelstrukturen einen Abstand von weniger als 1000 µm, weniger als 900 µm, weniger als 800 µm, weniger als 700 mm, weniger als 600 µm, mindestens weniger als 500 µm oder weniger als 400 µm aufweisen.

Die vorstehenden Maße können sich insbesondere auf eine Längsachse beziehungsweise Längsmittelachse der jeweiligen Nadelstruktur beziehen. Durch eine derartige geometrische Anordnung einer Mehrzahl von Mikronadeln kann eine dichte Nadelanordnung und damit eine verhältnismäßig große Wirkstoffverabreichung über ein verhältnismäßig kleines Hautareal gewährleistet werden. Die Anwenderfreundlichkeit einer solchen Mikronadelvorrichtung kann auf diese Weise weiter verbessert werden.

Ein weiterer unabhängiger Aspekt der vorliegenden Lehre betrifft ein medizinisches Pflaster, insbesondere zur transdermalen und/oder intradermalen Wirkstoffverabreichung. Ein solches Pflaster wird mit einer Mehrzahl von voranstehend beschriebenen Mikronadeln und/oder mit einer Mikronadel gemäß der voranstehenden Beschreibung ausgestattet. Des Weiteren kann ein solches Pflaster mit einer Klebevorrichtung, wie beispielsweise einer Klebstreifenschicht ausgestattet werden, über die die jeweiligen Mikronadeln beziehungsweise die Mikronadelvorrichtung fest auf der Haut aufgeklebt werden kann und eine sichere Wirkstoffverabreichung gewährleistet wird.

Die vorliegende Erfindung wird nachfolgend beispielhaft unter Bezugnahme auf die beigefügten Figuren näher erläutert.

Es zeigen jeweils schematisch:
- Fig. 1: eine Perspektivdarstellung einer Mikronadel gemäß einem ersten Ausführungsbeispiel der vorliegenden Erfindung,
- Fig. 2: eine Perspektivdarstellung einer Mikronadel gemäß einem weiteren Ausführungsbeispiel der vorliegenden Erfindung,
- Fig. 3A: eine Perspektivdarstellung einer Mikronadel gemäß einem noch weiteren Ausführungsbeispiel der vorliegenden Erfindung,
- Fig. 3B: ein Längsschnitt der Mikronadel gemäß Fig. 3A,
- Fig. 4A: eine Perspektivdarstellung einer Mikronadel gemäß einem noch weiteren Ausführungsbeispiel der vorliegenden Erfindung,
- Fig. 4B: ein Längsschnitt der Mikronadel gemäß Fig. 4A,
- Fig. 5: eine Perspektivdarstellung einer Mikronadelvorrichtung gemäß einem Ausführungsbeispiel der vorliegenden Erfindung und
- Fig. 6: eine Perspektivdarstellung eines medizinischen Pflasters gemäß einer Ausführungsform der vorliegenden Erfindung.

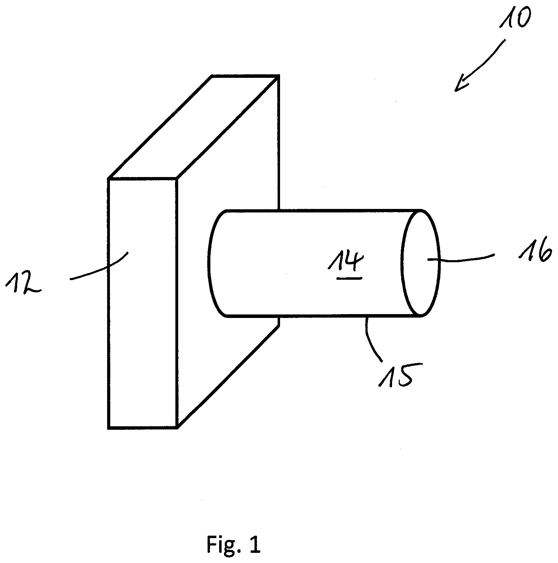

Die Fig. 1 zeigt eine Mikronadel 10 gemäß einem Ausführungsbeispiel der vorliegenden Erfindung. Die Mikronadel 10 weist eine Tragstruktur 12 und wenigstens eine an der Tragstruktur 12 angeordnete Nadelstruktur 14 zur Durchdringung der Hornzellschicht menschlicher und/oder tierischer Haut auf. Die Nadelstruktur 14 kann dabei insbesondere zur Durchdringung der Hornzellschicht menschlicher und/oder tierischer Haut dimensioniert sein beziehungsweise eine dazu geeignete geometrische Form aufweisen.

Die Nadelstruktur 14 wird erfindungsgemäß durch 3D-Siebdruck erzeugt. Hierzu wird die Nadelstruktur 14 schichtweise aufgebaut. Zwischen einzelnen Schritten zur schichtweisen Herstellung erfolgen Trocknungsschritte, die eine Trocknung der jeweils vorgedruckten Schicht gewährleisten.

Es ist ebenfalls möglich, dass die Tragstruktur 12 durch additive Fertigung, insbesondere 3D-Siebdruck, erzeugt ist. Die Nadelstruktur 14 und die Tragstruktur 12 können ferner einstückig ausgebildet sein und/oder durch eine ununterbrochene Verfahrensabfolge hergestellt sein. Insbesondere besteht die Möglichkeit, dass sowohl die Tragstruktur 12 als auch die Nadelstruktur 14 im Wege des 3D-Siebdrucks erzeugt sind und hierfür eine ununterbrochene Verfahrensabfolge des schichtweisen Aufbaus eingesetzt wird.

Ferner ist es möglich, dass die Tragstruktur 12 separat von der Nadelstruktur 14 erzeugt ist und dass die Nadelstruktur 14 mittels additiver Fertigung, insbesondere 3D-Siebdruck, an der Tragstruktur 12 angeordnet und/oder befestigt ist.

Wie der Fig. 1 entnommen werden kann, kann die Nadelstruktur 14 zumindest abschnittsweise zylindrisch ausgebildet sein beziehungsweise entlang ihrer Längserstreckung einen zumindest abschnittsweise gleichbleibenden und/oder kreisrunden Querschnitt aufweisen. Insbesondere kann die gesamte Nadelstruktur 14 zylindrisch ausgebildet sein beziehungsweise entlang ihrer Längserstreckung einen gleichbleibenden und/oder kreisrunden Querschnitt aufweisen.

Die Nadelstruktur 14 kann entlang ihrer Längserstreckung zumindest abschnittsweise einen Querschnittsdurchmesser von mindestens 30 µm, bevorzugt von mindestens 50 µm, bevorzugt von mindestens 70 µm, weiter bevorzugt von mindestens 80 µm oder von mehr als 90 µm aufweisen. Ebenso ist es möglich, dass die Nadelstruktur 14 entlang ihrer Längserstreckung zumindest abschnittsweise einen Querschnittsdurchmesser von mehr als 100 µm, noch weiter bevorzugt von mehr als 150 µm, noch weiter bevorzugt von mehr als 200 µm, noch weiter bevorzugt von mehr als 250 µm, noch weiter bevorzugt von mehr als 300 µm, aufweist.

Ferner kann die Nadelstruktur 14 entlang ihrer Längserstreckung zumindest abschnittsweise einen Querschnittsdurchmesser von weniger als 300 µm, bevorzugt von weniger als 250 µm, bevorzugt von weniger als 200 µm, weiter bevorzugt von weniger als 150 µm oder von weniger als 100 µm oder von weniger als 90 µm oder von weniger als 80 µm aufweisen.

Die Nadelstruktur 14 kann ferner eine Gesamtlänge von mindestens 200 µm, mindestens 300 µm, mindestens 400 µm, mindestens 500 µm, mindestens 600 µm, mindestens 700 µm oder weniger als 1000 µm, weniger als 900 µm, weniger als 800 µm, weniger als 700 µm, weniger als 600 µm, weniger als 500 µm oder weniger als 400 µm aufweisen. Die Länge der Nadelstruktur 14 kann sich dabei insbesondere zwischen der Tragstruktur 12 und einem von der Tragstruktur 12 abgewandten freien Ende 16 erstrecken.

In dem Ausführungsbeispiel gemäß Fig. 1 kann die Nadelstruktur 14 eine entlang der Längserstreckung gleichbleibende Querschnittsform und Querschnittsgröße aufweisen. Der Außenumfang 15 der Nadelstruktur 14 bleibt demnach entlang der Längserstreckung unveränderlich. Eine solche geometrische Ausgestaltung lässt sich im Wege der additiven Fertigung, insbesondere mittels 3D-Siebdruck, mit nur geringem Aufwand erzeugen.

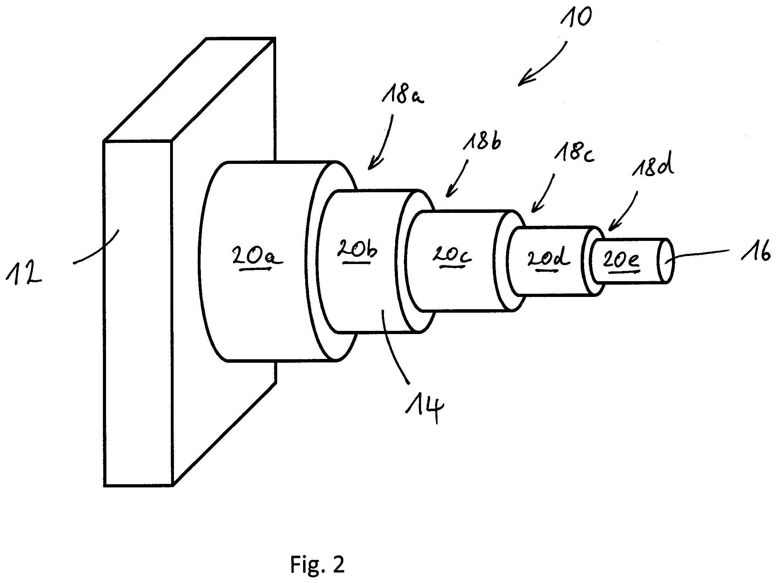

Die Fig. 2 zeigt eine weitere Ausführungsform einer Mikronadel 10 gemäß der vorliegenden Erfindung. Die Mikronadel 10 gemäß Fig. 2 unterscheidet sich von der Ausführungsform in Fig. 1 hinsichtlich der geometrischen Ausgestaltung der Nadelstruktur 14. So weist die Nadelstruktur 14 in Fig. 2 entlang ihrer Längserstreckung einen sich verändernden Querschnitt auf. Insbesondere weist die Nadelstruktur 14 in Fig. 2 entlang ihrer Längserstreckung unterschiedliche Querschnittsgrößen auf. Hierzu kann die Nadelstruktur 14 entlang ihrer Längserstreckung beispielsweise gestuft ausgebildet sein.

In dem Ausführungsbeispiel gemäß Fig. 2 sind rein beispielhaft vier Stufen 18a, 18b, 18c und 18d vorgesehen. Zwischen den Stufen 18a und 18b, 18b und 18c sowie 18c und 18d kann die Nadelstruktur 14 einen gleichbleibenden Querschnitt aufweisen beziehungsweise eine entlang der Längserstreckung kontinuierliche Querschnittsform aufweisen. Ebenso kann die Querschnittsgröße zwischen zwei Stufen 18a und 18b oder 18b und 18c oder 18c und 18d gleichbleibend ausgestaltet sein. Schließlich kann die Querschnittsgröße und/oder Querschnittsform zwischen der Tragstruktur 12 und der Stufe 18a und/oder zwischen der Stufe 18d und dem freien Ende 16 gleichbleibend ausgebildet sein.

Die Stufen 18a, 18b, 18c und 18d können die Nadelstruktur 14 in insgesamt fünf Nadelstrukturabschnitte 20a, 20b, 20c, 20d und 20e unterteilen. Dabei grenzt der Nadelstrukturabschnitt 20a an die Tragstruktur 12 an und der Nadelstrukturabschnitt 20e bildet das freie Ende 16. Die Nadelstrukturabschnitte 20a bis 20e können jeweils eine gleiche Querschnittsform aufweisen, jedoch unterschiedliche Querschnittsgrößen. Mit zunehmendem Abstand von der Tragstruktur 12 kann sich der jeweilige Querschnittsdurchmesser der einzelnen Nadelstrukturabschnitte 20a bis 20e verkleinern. Demgemäß kann sich die Querschnittsgröße der Nadelstrukturabschnitte 20a bis 20e stufenweise ausgehend von der Tragstruktur 12 verkleinern.

Die voranstehend bezüglich der Ausführungsform in Fig. 1 erwähnten Abmessungen können auch für die einzelnen Nadelstrukturabschnitte 20a bis 20e gelten. Ferner können sich die Angaben hinsichtlich der Gesamtlänge der Nadelstruktur 14 in Fig. 2 auf die Summe der Einzellängen der Nadelstrukturabschnitte 20a bis 20e beziehen.

Die Länge eines sich zwischen zwei Stufen 18a und 18b, 18b und 18c sowie 18c und 18d erstreckenden Nadelstrukturabschnitt 20b, 20c sowie 20d kann eine Länge von weniger als 200 µm, weniger als 150 µm, weniger als 100 µm oder weniger als 50 µm aufweisen. Ein solcher Nadelstrukturabschnitt kann ferner eine Länge von mindestens 20 µm, mindestens 50 µm, mindestens 100 µm, mindestens 150 µm, mindestens 200 µm oder mindestens 250 µm aufweisen. Die voranstehenden Maßangaben können ferner auch für den Nadelstrukturabschnitt 20a, der sich zwischen der Tragstruktur 12 und der Stufe 18a erstreckt. Ebenso können sich die voranstehenden Maßangaben für den Nadelstrukturabschnitt 20e gelten, der sich zwischen der Stufe 18d und dem freien Ende 16 erstreckt.

Die Nadelstruktur 14 gemäß den Fig. 1 und 2 kann wenigstens einen Wirkstoff aufweisen. Ebenso kann die Nadelstruktur 14 unterschiedliche Wirkstoffe aufweisen. Die Wirkstoffe beziehungsweise Wirkstoffdichten der Nadelstruktur 14 können entlang der Längserstreckung unterschiedlich sein beziehungsweise entlang der Längserstreckung variieren.

Ferner kann die Nadelstruktur 14 gemäß den Fig. 1 und 2 zur Wirkstoffverabreichung durch Materialauflösung ausgebildet sein. Insbesondere besteht die Möglichkeit, dass sich die Nadelstruktur 14 zur Wirkstoffverabreichung vollständig auflöst. Das Entfernen der Nadelstruktur 14 im Anschluss an die Wirkstoffverabreichung ist somit entbehrlich.

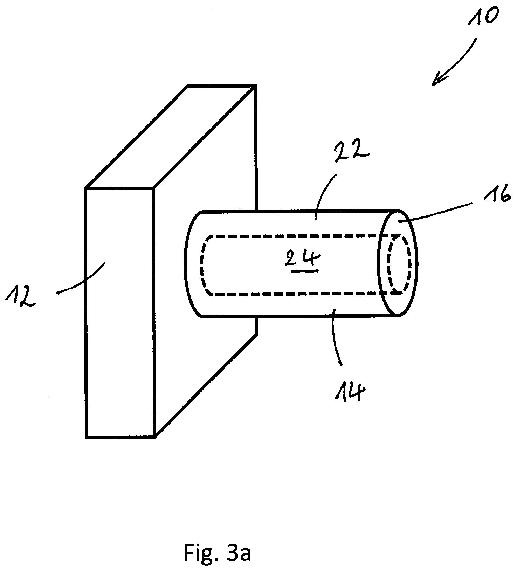

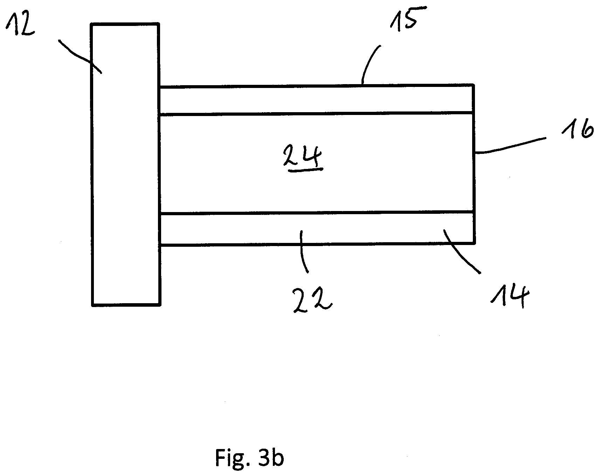

Die Fig. 3A und 3B zeigen eine weitere Ausführungsform einer Mikronadel 10 gemäß der vorliegenden Erfindung. Die Ausführungsform der Fig. 3 unterscheidet sich von der Ausführungsform in Fig. 1 dadurch, dass die Nadelstruktur 14 eine zur Auflösung ausgebildete Beschichtung mit wenigstens einem Wirkstoff aufweist. Die Beschichtung 22 kann dabei auf einer Kernstruktur 24 ausgebildet beziehungsweise auf dieser oder um diese herum angeordnet sein. Es ist ferner möglich, dass die Kernstruktur 24 nicht zur Auflösung ausgebildet ist beziehungsweise aus einem auflösungsresistenten Material gebildet ist, das sich von dem Material der Beschichtung 22 unterscheidet.

Ferner ist es möglich, dass sich sowohl die Beschichtung 22 als auch die Kernstruktur 24 zur Wirkstoffverabreichung durch Auflösung ausgebildet sind, wobei die Beschichtung 22 einen anderen Wirkstoff oder andere Wirkstoffe enthält als die Kernstruktur 24, so dass ein jeweils erwünschtes Wirkstoffverabreichungsprofil erzielt werden kann. Die voranstehenden Maßangaben hinsichtlich des Querschnittsdurchmessers können sich bei der Ausführungsform in Fig. 3 auf den Gesamtquerschnittsdurchmesser beziehen, der durch die Kernstruktur 24 sowie die Beschichtung 22 gebildet wird.

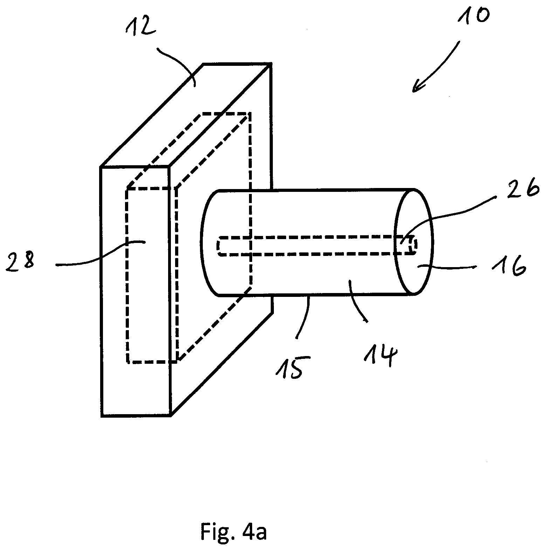

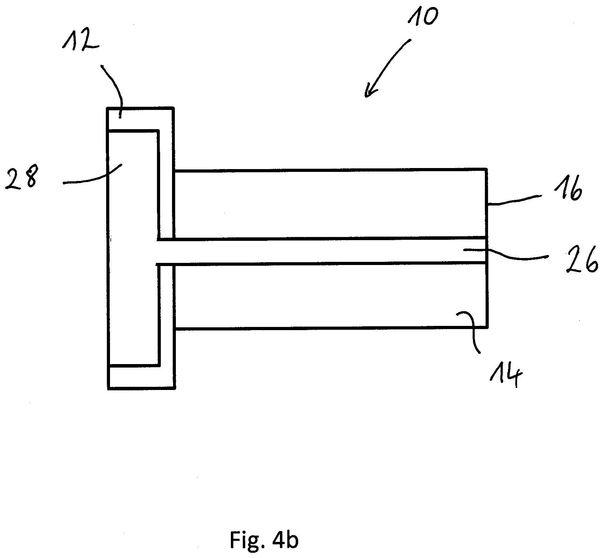

Die Fig. 4A und 4B zeigen eine weitere Ausführungsform einer Mikronadel 10 gemäß der vorliegenden Erfindung. Die Ausführungsform in den Fig. 4A und 4B unterscheidet sich von der Ausführungsform in Fig. 1 dadurch, dass die Nadelstruktur 14 einen Hohlraum 26 mit wenigstens einem darin angeordneten Wirkstoff aufweist. Dementsprechend kann die Nadelstruktur 12 gemäß Fig. 4A und 4B zur Wirkstoffverabreichung aus dem Hohlraum 26 der Nadelstruktur 14 ausgebildet sein.

Bei dem Hohlraum 26 kann es sich um einen entlang der Längserstreckung verlaufenden Kanal handeln, der in dem freien Ende 16 der Nadelstruktur 14 mündet. Der Hohlraum 26 kann ferner bis in ein Wirkstoffreservoir 28 verlaufen, welches zumindest teilweise durch die Tragstruktur 12 gebildet ist beziehungsweise in der Tragstruktur 12 eingelassen ist. Es ist ferner möglich, dass das Wirkstoffreservoir 28 lediglich innerhalb der Nadelstruktur 14 ausgebildet ist, was hier nicht näher dargestellt ist.

Nach Durchdringung der Hornzellschicht menschlicher und/oder tierischer Haut kann aus dem Hohlraum 26 eine Wirkstoffverabreichung in den jeweiligen Organismus erfolgen, wobei hierbei auch eine Entleerung beziehungsweise eine teilweise Entleerung des Wirkstoffreservoirs 28 realisiert werden kann. Im Übrigen kann die Nadelstruktur 14 resistent gegen Auflösung in einem lebenden Organismus ausgebildet sein. Ebenso ist es möglich, dass die Nadelstruktur 14 gemäß Fig. 4 ebenfalls zur Wirkstoffverabreichung durch Auflösung ausgebildet ist. Hierbei kann die Auflösung der Nadelstruktur 14 im Anschluss an die Wirkstoffverabreichung aus dem Hohlraum 26 erfolgen, so dass beispielsweise in zeitlicher Abfolge unterschiedliche Wirkstoffe in dem jeweiligen Organismus freigesetzt werden können.

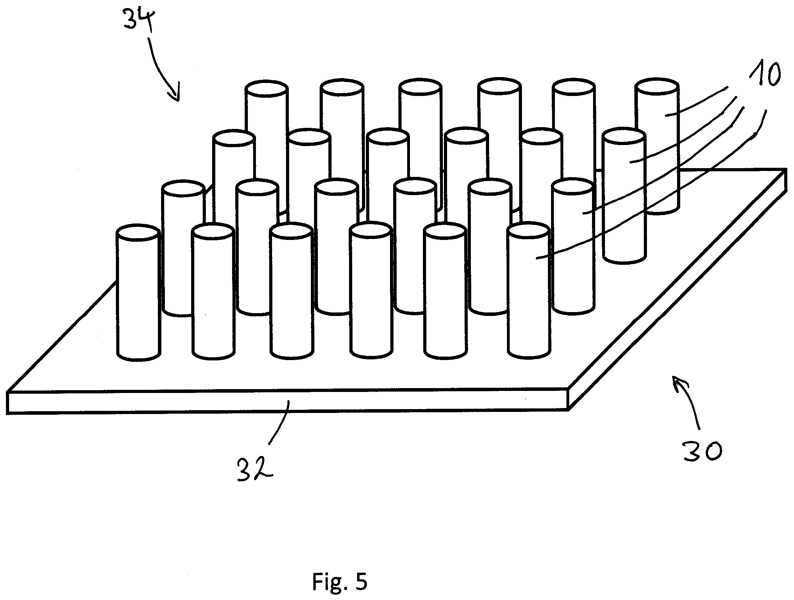

Die Fig. 5 zeigt eine Mikronadelvorrichtung 30 gemäß einer Ausführungsform der vorliegenden Erfindung. Die Mikronadelvorrichtung 30 weist eine Vielzahl von Mikronadeln 10 gemäß Fig. 1 auf. Ebenso ist es möglich, dass die Mikronadelvorrichtung 30 aus Mikronadeln 10 gemäß einem der weiteren Ausführungsbeispiele in den Fig. 2 bis 4 gebildet ist, was hier jedoch nicht näher dargestellt ist.

Gemäß Fig. 5 sind die einzelnen Mikronadeln 10 innerhalb der Mikronadelvorrichtung 30 in einer bestimmten Anordnung zueinander vorgesehen beziehungsweise bilden ein vordefiniertes oder auch zufälliges Anordnungsmuster. Dabei können die Tragstrukturen 12 der Mikronadeln 10 miteinander verbunden beziehungsweise einstückig ausgebildet sein. Die Tragstrukturen 12 der Mikronadeln 10 können somit eine Gesamttragstruktur 32 bilden, an der die einzelnen Nadelstrukturen 12 angeordnet beziehungsweise befestigt sind.

Die Nadelstrukturen 12 der einzelnen Mikronadeln 10 können zueinander einen definierten Abstand aufweisen. Rein beispielhaft können die Nadelstrukturen 12 von zwei unmittelbar zueinander benachbarten Mikronadeln 10 einen Abstand von mehr als 300 µm oder weniger als 500 µm, rein beispielhaft etwa 350 µm aufweisen. Die Anzahl der einzelnen Mikronadeln 10 einer Mikronadelvorrichtung 30 kann in Abhängigkeit der jeweiligen Anwendung beliebig variiert beziehungsweise ausgewählt werden. Die Mikronadeln 10 der Mikronadelvorrichtung 30 bilden somit ein Nadelarray 34.

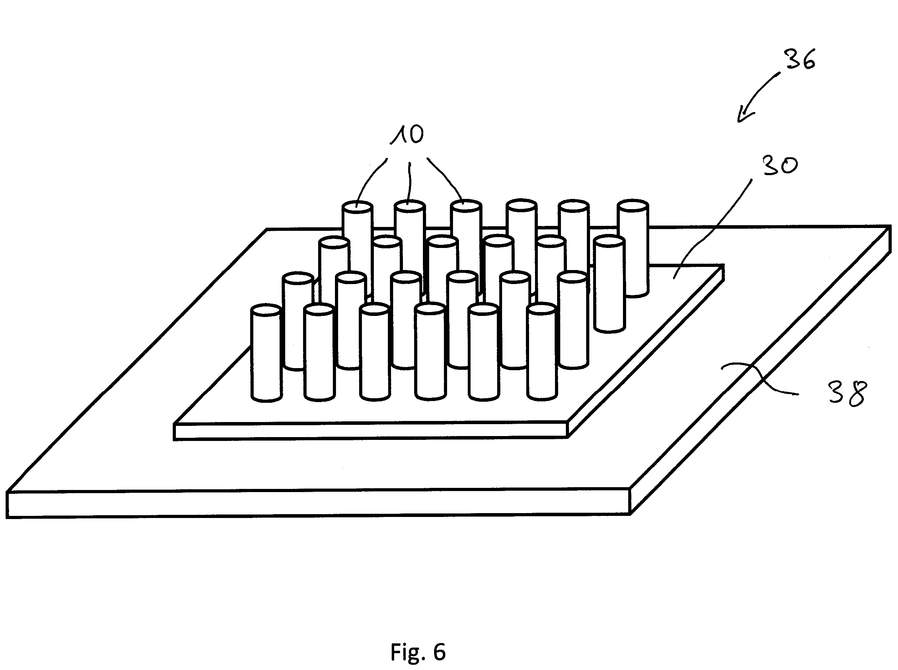

Die Fig. 6 zeigt ein medizinisches Pflaster 36. Ein solches medizinisches Pflaster 36 kann eine Mikronadelvorrichtung 30 beziehungsweise eine Mehrzahl von Mikronadeln 10 aufweisen. Die Mikronadelvorrichtung 30 beziehungsweise die Mikronadeln 10 können dabei auf einem Klebeband 38 beziehungsweise medizinischem Tapematerial angeordnet beziehungsweise befestigt sein. Das Klebeband 38 eignet sich insbesondere zur klebenden Befestigung auf menschlicher oder tierischer Haut, wodurch eine über einen Zeitraum erfolgende Wirkstoffverabreichung durch die Mikronadeln 10 mit hoher Sicherheit gewährleistet werden kann.

### Bezugszeichenliste

- 10: Mikronadel
- 12: Tragstruktur
- 14: Nadelstruktur
- 15: Außenumfang
- 16: freies Ende
- 18a-18d: Stufen
- 20a-20e: Nadelstrukturabschnitte
- 22: Beschichtung
- 24: Kernstruktur
- 26: Hohlraum
- 28: Wirkstoffreservoir
- 30: Mikronadelvorrichtung
- 32: Gesamttragstruktur
- 34: Nadelarray
- 36: medizinisches Pflaster
- 38: Klebeband

## Patentansprüche

1. Verfahren zur Herstellung einer Mikronadel (10), insbesondere zur transdermalen und/oder intradermalen Wirkstoffverabreichung, bei dem eine Tragstruktur (12) bereitgestellt wird und bei dem wenigstens eine an der Tragstruktur (12) angeordnete Nadelstruktur (14) zur Durchdringung der Hornzellschicht menschlicher und/oder tierischer Haut durch 3D-Siebdruck schichtweise erzeugt wird, wobei zwischen einzelnen Schritten zur schichtweisen Erzeugung der Nadelstruktur (14) Trocknungsschritte erfolgen, durch die eine Trocknung der jeweils vorgedruckten Schicht gewährleistet wird.

2. Verfahren (10) nach Anspruch 1, **dadurch gekennzeichnet, dass** die Tragstruktur (12) durch additive Fertigung, insbesondere 3D-Siebdruck, erzeugt wird und/oder dass die Nadelstruktur (14) und die Tragstruktur (12) einstückig ausgebildet werden und/oder durch eine ununterbrochene Verfahrensabfolge hergestellt werden.

3. Verfahren (10) nach Anspruch 1, **dadurch gekennzeichnet, dass** die Tragstruktur (12) separat von der Nadelstruktur (14) erzeugt wird und/oder dass die Nadelstruktur (14) mittels 3D-Siebdruck an der Tragstruktur (12) angeordnet und/oder befestigt wird.

4. Verfahren (10) nach einem der vorstehenden Ansprüche, **dadurch gekennzeichnet, dass** die Nadelstruktur (14) zumindest abschnittsweise zylindrisch ausgebildet wird und/oder mit einem entlang ihrer Längserstreckung zumindest abschnittsweise gleichbleibenden und/oder kreisrunden Querschnitt ausgebildet wird und/oder dass die gesamte Nadelstruktur (14) zylindrisch ausgebildet wird und/oder mit einem entlang ihrer Längserstreckung gleichbleibenden und/oder kreisrunden Querschnitt ausgebildet wird.

5. Verfahren (10) nach einem der vorstehenden Ansprüche, **dadurch gekennzeichnet, dass** die Nadelstruktur (14) mit einem entlang ihrer Längserstreckung sich verändernden Querschnitt ausgebildet wird und/oder mit unterschiedlichen Querschnittsgrößen und/oder gleichbleibenden Querschnittsformen ausgebildet wird.

6. Verfahren (10) nach einem der vorstehenden Ansprüche, **dadurch gekennzeichnet, dass** die Nadelstruktur (14) entlang ihrer Längserstreckung gestuft ausgebildet wird und/oder mit einem zwischen wenigstens zwei Stufen (18) gleichbleibenden Querschnitt, insbesondere mit einer gleichbleibenden Querschnittsform und/oder Querschnittsgröße ausgebildet wird.

7. Verfahren (10) nach einem der vorstehenden Ansprüche, **dadurch gekennzeichnet, dass** die Nadelstruktur (14) entlang ihrer Längserstreckung zumindest abschnittsweise mit einem Querschnittsdurchmesser von mindestens 30 µm, bevorzugt von mindestens 50 µm, bevorzugt von mindestens 70 µm, weiter bevorzugt von mindestens 80 µm oder von mehr als 90 µm, insbesondere mehr als 100 µm, noch weiter bevorzugt von mehr als 150 µm, noch weiter bevorzugt von mehr als 200 µm, noch weiter bevorzugt von mehr als 250 µm, noch weiter bevorzugt von mehr als 300 µm, ausgebildet wird.

8. Verfahren (10) nach einem der vorstehenden Ansprüche, **dadurch gekennzeichnet, dass** die Nadelstruktur (14) entlang ihrer Längserstreckung zumindest abschnittsweise mit einem Querschnittsdurchmesser von weniger als 300 µm, bevorzugt von weniger als 250 µm, bevorzugt von weniger als 200 µm, weiter bevorzugt von weniger als 150 µm oder von weniger als 100 µm oder von weniger als 90 µm oder von weniger als 80 µm ausgebildet wird.

9. Verfahren (10) nach einem der vorstehenden Ansprüche, **dadurch gekennzeichnet, dass** die Nadelstruktur (14) mit einer Gesamtlänge von mindestens 200 µm, mindestens 300 µm, mindestens 400 µm, mindestens 500 µm, mindestens 600 µm, mindestens 700 µm oder weniger als 1000 µm, weniger als 900 µm, weniger als 800 µm, weniger als 700 µm, weniger als 600 µm, weniger als 500 µm oder weniger als 400 µm ausgebildet wird und/oder dass sich die Länge der Nadelstruktur (14) zwischen der Tragstruktur (12) und einem von der Tragstruktur (12) abgewandten freien Ende (16) erstreckt.

10. Verfahren (10) nach einem der vorstehenden Ansprüche, **dadurch gekennzeichnet, dass** die Nadelstruktur (14) mit zumindest einem sich in Längsrichtung zwischen zwei Stufen (18) erstreckenden Nadelstrukturabschnitt (20) mit einer Länge von weniger als 200 µm, weniger als 150 µm, weniger als 100 µm oder weniger als 50 µm ausgebildet wird.

11. Verfahren (10) nach einem der vorstehenden Ansprüche, **dadurch gekennzeichnet, dass** die Nadelstruktur (14) mit wenigstens einem sich in Längsrichtung zwischen zwei Stufen (18) erstreckenden Nadelstrukturabschnitt (20) mit einer Länge von mindestens 20 µm, mindestens 50 µm, mindestens 100 µm, mindestens 150 µm, mindestens 200 µm oder mindestens 250 µmausgebildet wird.

12. Verfahren (10) nach einem der vorstehenden Ansprüche, **dadurch gekennzeichnet, dass** die Nadelstruktur (14) mit wenigstens einem Wirkstoff ausgebildet wird und/oder dass die Nadelstruktur (14) mit mehreren Wirkstoffen ausgebildet wird und/oder dass die Nadelstruktur (14) mit entlang ihrer Längserstreckung unterschiedlichen Wirkstoffdichten und/oder Wirkstoffen ausgebildet wird und/oder dass die Nadelstruktur (14) zur Wirkstoffverabreichung durch Materialauflösung ausgebildet wird.

13. Verfahren (10) nach einem der vorstehenden Ansprüche, **dadurch gekennzeichnet, dass** die Nadelstruktur (14) mit einer zur Auflösung ausgebildeten Beschichtung (22) mit wenigstens einem Wirkstoff ausgebildet wird und/oder dass die Nadelstruktur (14) mit einem Hohlraum (26) mit wenigstens einem darin angeordneten Wirkstoff ausgebildet wird und/oder dass die Nadelstruktur (14) zur Wirkstoffverabreichung aus einem Hohlraum (26) der Nadelstruktur (14) ausgebildet wird.

14. Verfahren zur Herstellung einer Mikronadelvorrichtung (30), insbesondere zur transdermalen und/oder intradermalen Wirkstoffverabreichung, bei dem eine Mehrzahl von Mikronadeln (10) nach einem Verfahren gemäß einem der vorstehenden Ansprüche erzeugt werden, wobei die Mikronadeln (10) bevorzugt als Nadelarray (34) ausgebildet werden.

15. Verfahren nach Anspruch 14, **dadurch gekennzeichnet, dass** die Tragabschnitte (12) der Mikronadeln (14) einstückig miteinander ausgebildet oder zu einer Gesamttragstruktur (32) verbunden werden.

## Claims

1. Method for producing a microneedle (10), in particular for transdermal and/or intradermal administration of active substances, in which a support structure (12) is provided and in which at least one needle structure (14), arranged on the support structure (12), for penetrating the horny layer of human and/or animal skin is produced layer by layer by means of 3D screen printing, wherein drying steps are carried out between individual steps for the layer-by-layer production of the needle structure (14), thereby ensuring that the respective pre-printed layer is dried.

2. Method (10) according to claim 1, **characterized in that** the support structure (12) is produced by additive manufacturing, in particular 3D screen printing, and/or that the needle structure (14) and the support structure (12) are formed in one piece and/or are produced by an uninterrupted sequence of processes.

3. Method (10) according to claim 1, **characterized in that** the support structure (12) is produced separately from the needle structure (14) and/or that the needle structure (14) is arranged and/or attached to the support structure (12) by means of 3D screen printing.

4. Method (10) according to one of the preceding claims, **characterized in that** the needle structure (14) is formed cylindrically at least in sections and/or is formed with a cross-section that is at least sectionally uniform and/or circular along its longitudinal extension and/or that the entire needle structure (14) is designed to be cylindrical and/or has a cross-section that is uniform and/or circular along its longitudinal extension.

5. Method (10) according to one of the preceding claims, **characterized in that** the needle structure (14) is formed with a cross-section that varies along its longitudinal extension and/or with different cross-sectional sizes and/or uniform cross-sectional shapes.

6. Method (10) according to one of the preceding claims, **characterized in that** the needle structure (14) is formed in steps along its longitudinal extension and/or with a cross-section that remains constant between at least two steps (18), in particular with a constant cross-sectional shape and/or cross-sectional size.

7. Method (10) according to one of the preceding claims, **characterized in that** the needle structure (14) is formed along its longitudinal extension at least in sections with a cross-sectional diameter of at least 30 µm, preferably at least 50 µm, preferably at least 70 µm, more preferably of at least 80 µm or of more than 90 µm, in particular more than 100 µm, even more preferably of more than 150 µm, even more preferably of more than 200 µm, even more preferably of more than 250 µm, even more preferably of more than 300 µm.

8. Method (10) according to one of the preceding claims, **characterized in that** the needle structure (14) is formed along its longitudinal extension at least in sections with a cross-sectional diameter of less than 300 µm, preferably less than 250 µm, preferably less than 200 µm, more preferably less than 150 µm or less than 100 µm or less than 90 µm or less than 80 µm.

9. Method (10) according to one of the preceding claims, **characterized in that** the needle structure (14) is formed with a total length of at least 200 µm, at least 300 µm, at least 400 µm, at least 500 µm, at least 600 µm, at least 700 µm or less than 1000 µm, less than 900 µm, less than 800 µm, less than 700 µm, less than 600 µm, less than 500 µm or less than 400 µm, and/or that the length of the needle structure (14) extends between the support structure (12) and a free end (16) facing away from the support structure (12).

10. Method (10) according to one of the preceding claims, **characterized in that** the needle structure (14) is formed with at least one needle structure section (20) extending longitudinally between two steps (18) with a length of less than 200 µm, less than 150 µm, less than 100 µm or less than 50 µm.

11. Method (10) according to one of the preceding claims, **characterized in that** the needle structure (14) is formed with at least one needle structure section (20) extending longitudinally between two steps (18) with a length of at least 20 µm, at least 50 µm, at least 100 µm, at least 150 µm, at least 200 µm or at least 250 µm.

12. Method (10) according to one of the preceding claims, **characterized in that** the needle structure (14) is formed with at least one active substance and/or that the needle structure (14) is formed with several active substances and/or that the needle structure (14) is formed with different active substance densities and/or active substances along its longitudinal extension and/or that the needle structure (14) is formed for active substance administration by material dissolution.

13. Method (10) according to one of the preceding claims, **characterized in that** the needle structure (14) is formed with a coating (22) designed to dissolve, with at least one active ingredient, and/or that the needle structure (14) is formed with a cavity (26) with at least one active substance arranged therein and/or that the needle structure (14) is formed for administering active substances from a cavity (26) of the needle structure (14).

14. Method for manufacturing a microneedle device (30), in particular for transdermal and/or intradermal active substance administration, in which a plurality of microneedles (10) are produced according to a method according to one of the preceding claims, wherein the microneedles (10) are preferably formed as a needle array (34).

15. Method according to claim 14, **characterized in that** the support sections (12) of the microneedles (14) are formed integrally with one another or are connected to form an overall support structure (32).

## Revendications

1. Procédé pour la fabrication d'une microaiguille (10), en particulier pour l'administration transdermique et/ou intradermique de substances actives, dans lequel une structure de support (12) est fournie et dans lequel au moins une structure d'aiguille (14) disposée sur la structure de support (12) et permettant la pénétration de la couche de cornéocytes de la peau humaine et/ou animale est produite par couches par sérigraphie 3D, dans lequel des étapes de séchage sont effectuées entre des étapes individuelles pour la production par couches de la structure d'aiguille (14), par lesquelles étapes de séchage un séchage de la couche respectivement préimprimée est garanti.

2. Procédé (10) selon la revendication 1, **caractérisé en ce que** la structure de support (12) est produite par fabrication additive, en particulier par sérigraphie 3D, **et/ou en ce que** la structure d'aiguille (14) et la structure de support (12) sont réalisées d'une seule pièce et/ou sont fabriquées par une succession ininterrompue de procédés.

3. Procédé (10) selon la revendication 1, **caractérisé en ce que** la structure de support (12) est produite séparément de la structure d'aiguille (14) **et/ou en ce que** la structure d'aiguille (14) est disposée et/ou fixée sur la structure de support (12) par sérigraphie 3D.

4. Procédé (10) selon l'une des revendications précédentes, **caractérisé en ce que** la structure d'aiguille (14) est réalisée sous forme cylindrique au moins dans certaines sections et/ou est réalisée avec une section transversale constante et/ou circulaire au moins dans certaines sections le long de son extension longitudinale **et/ou en ce que** l'ensemble de la structure d'aiguille (14) est réalisé sous forme cylindrique et/ou est réalisé avec une section transversale constante et/ou circulaire le long de son extension longitudinale.

5. Procédé (10) selon l'une des revendications précédentes, **caractérisé en ce que** la structure d'aiguille (14) est réalisée avec une section transversale qui varie le long de son extension longitudinale et/ou est réalisée avec des tailles de section transversale différentes et/ou des formes de section transversale constantes.

6. Procédé (10) selon l'une des revendications précédentes, **caractérisé en ce que** la structure d'aiguille (14) est réalisée en gradins le long de son extension longitudinale et/ou est réalisée avec une section transversale constante entre au moins deux gradins (18), en particulier avec une forme de section transversale et/ou une taille de section transversale constantes.

7. Procédé (10) selon l'une des revendications précédentes, **caractérisé en ce que** la structure d'aiguille (14) est réalisée, au moins dans certaines sections le long de son extension longitudinale, avec un diamètre de section transversale d'au moins 30 µm, de préférence d'au moins 50 µm, de préférence d'au moins 70 µm, plus préférablement d'au moins 80 µm ou de plus de 90 µm, en particulier de plus de 100 µm, encore plus préférablement de plus de 150 µm, encore plus préférablement de plus de 200 µm, encore plus préférablement de plus de 250 µm, encore plus préférablement de plus de 300 µm.

8. Procédé (10) selon l'une des revendications précédentes, **caractérisé en ce que** la structure d'aiguille (14) est réalisée, au moins dans certaines sections le long de son extension longitudinale, avec un diamètre de section transversale de moins de 300 µm, de préférence de moins de 250 µm, de préférence de moins de 200 µm, plus préférablement de moins de 150 µm ou de moins de 100 µm ou de moins de 90 µm ou de moins de 80 µm.

9. Procédé (10) selon l'une des revendications précédentes, **caractérisé en ce que** la structure d'aiguille (14) est réalisée avec une longueur totale d'au moins 200 µm, d'au moins 300 µm, d'au moins 400 µm, d'au moins 500 µm, d'au moins 600 µm, d'au moins 700 µm ou de moins de 1000 µm, de moins de 900 µm, de moins de 800 µm, de moins de 700 µm, de moins de 600 µm, de moins de 500 µm ou de moins de 400 µm, **et/ou en ce que** la longueur de la structure d'aiguille (14) s'étend entre la structure de support (12) et une extrémité libre (16) opposée à la structure de support (12).

10. Procédé (10) selon l'une des revendications précédentes, **caractérisé en ce que** la structure d'aiguille (14) est réalisée avec au moins une section de structure d'aiguille (20) s'étendant dans la direction longitudinale entre deux gradins (18) et comportant une longueur inférieure à 200 µm, inférieure à 150 µm, inférieure à 100 µm ou inférieure à 50 µm.

11. Procédé (10) selon l'une des revendications précédentes, **caractérisé en ce que** la structure d'aiguille (14) est réalisée avec au moins une section de structure d'aiguille (20) s'étendant dans la direction longitudinale entre deux gradins (18) et comportant une longueur d'au moins 20 µm, d'au moins 50 µm, d'au moins 100 µm, d'au moins 150 µm, d'au moins 200 µm ou d'au moins 250 µm.

12. Procédé (10) selon l'une des revendications précédentes, **caractérisé en ce que** la structure d'aiguille (14) est réalisée avec au moins une substance active **et/ou en ce que** la structure d'aiguille (14) est réalisée avec plusieurs substances actives **et/ou en ce que** la structure d'aiguille (14) est réalisée avec des densités de substances actives et/ou des substances actives différentes le long de son extension longitudinale **et/ou en ce que** la structure d'aiguille (14) est réalisée pour l'administration de substances actives par dissolution de matière.

13. Procédé (10) selon l'une des revendications précédentes, **caractérisé en ce que** la structure d'aiguille (14) est réalisée avec un revêtement (22) réalisé pour la dissolution comportant au moins une substance active **et/ou en ce que** la structure d'aiguille (14) est réalisée avec une cavité (26) comportant au moins une substance active disposée dans celle-ci **et/ou en ce que** la structure d'aiguille (14) est réalisée pour l'administration de substances actives à partir d'une cavité (26) de la structure d'aiguille (14).

14. Procédé pour la fabrication d'un dispositif à microaiguilles (30), en particulier pour l'administration transdermique et/ou intradermique de substances actives, dans lequel une pluralité de microaiguilles (10) sont produites selon un procédé conformément à l'une des revendications précédentes, dans lequel les microaiguilles (10) sont de préférence réalisées sous forme de réseau d'aiguilles (34).

15. Procédé selon la revendication 14, **caractérisé en ce que** les sections de support (12) des microaiguilles (14) sont réalisées d'une seule pièce les unes avec les autres ou sont reliées pour former une structure de support globale (32).
